# EUROPEAN PATENT APPLICATION

(11) **EP 1 420 252 A2**
(43) Date of publication of application: **19.05.2004**
(21) Application number: 03079061.2
(22) Date of filing: 24.12.1998
(51) Int. Cl.: G01N 33/543, C12Q 1/68, B01J 19/00, G01N 35/00

(54) **Detection and/or quantification method of a target molecule by binding with a capture molecule fixed on the surface of a disc**

(30) Priority: 30.12.1997 US 71726 P
(62) Divisional of application: 98965057.7
(71) Applicant: Remacle, José, 5020 Malonne (BE)
(72) Inventor: Remacle, José, 5020 Malonne (BE)
(74) Representative: Van Malderen, Joelle

(57) **Abstract**

The present invention is related to a method for the detection and/or the quantification of a target molecule by its binding with a non-cleavable capture molecule fixed on the surface of a disc comprising registered data.

The present invention is also related to a disc having fixed upon its surface a non-cleavable capture molecule, to its preparation process, and to a diagnostic and/or reading device of said disc or comprising said disc.

## Description

### Field of the invention

The present invention is related to a detection and/or quantification method of a target molecule by its binding with a capture molecule fixed on the surface of a disc.

The present invention is also related to a disc having fixed upon its surface a non-cleavable capture molecule, to its preparation process, and to a diagnostic and/or reading device of said disc or comprising said disc.

### Background of the invention

The complete detection process of a target molecule (like a nucleotide sequence obtained from a microorganism) requires the steps of :
- possibly a preparation of the sample,
- possibly an amplification of the "purified" molecule,
- a binding of said molecule on a "capture" molecule (i.e. sequence or receptor) preferably fixed on a solid support,
- its labeling, and finally
- the analysis of the obtained signal from said labeling.

Therefore, it exists a need for a possibly simplified automatic device and method that could perform several (or possibly all) of these steps, especially the analysis of the obtained signal, and could discriminate among a large number of complex molecules the specific molecule or microorganism to be detected.

The search for miniaturization of molecules binding on a determined small surface and identification of their location led to the use of microelectronic circuits for the construction of network designed as DNA-chips (US patent 5 632 957, US patent 5 605 662 and WO94/22889).

Another approach for the analysis of great numbers of biological molecules is the biochips. These biological molecules can be formed either by direct synthesis of capture molecules on the surface of biochips (WO97/29212) or by fixation of these capture molecules after their synthesis or isolation (WO98/28444). One of the drawbacks of said technology is the use of complicated and expensive instrumentation for reading out very low signals. In addition, this instrumentation should comprise specific software for the identification of the localization of the spots and for the integration of said signals when quantitative assays have to be performed.

Electronic devices can control the transport and attachment of specific binding entities like captured oligonucleotides or antibodies into specific micro-locations in order to make chips self addressable devices. For the fixation of molecules, one micro-electrode is positively charged to attract oligonucleotides on its surface. After binding with target molecules like DNA or antigens, detection is performed using epifluorescent type microscopic detection system for the localization of the microelectrode on which target molecules have been bound.

Said technique has been adapted for DNA binding proteins attached to substrates so as to form a network on this substrate resulting in a microcircuit (US patent 5,561,071).

However, there is a limit in the number of captured molecules that can be used due to the fact that they have to fix such capture molecules one by one on each of the micro-electrodes of the device. The other limitation is the resolution obtained in the discrimination between signals coming from adjacent electrodes, due to the dispersion of the emitted signal. Finally, the emission signal has to be analyzed and interpreted before being processed.

Therefore, it is necessary to provide upon a solid support an increased number of "capture molecules" in order to allow the identification and/or the quantification of specific target molecules, target microorganisms or portions thereof. However, classical detection supports and means can not be easily adapted for such applications, because they are limited by the number of "capture molecules" they may include, the discrimination between the signals and the specificity or reproducibility of the assay (see US 5 567 294). Moreover, the reading machines are too sophisticated and expensive.

The document WO98/01533 describes a cleavable signal element comprising a cleavable spacer having a substrate-attaching end (which can be a compact-disk), a signal-responsive end (which can be linked to a metallic beads, especially gold beads), and a first side member adapted to bind a first site on a chosen analyte and a second side member adapted to bind a second site of said chosen analyte. The signal is measured when the analyte is fixed upon the first side member and the second side member. Thereafter, the spacer is cleaved and the fixation of the analyte allows the detection of a positive signal.

However, this complex and expensive detection method and device is submitted to various false positives or false negatives in the detection of various complex analytes, which could develop various interactions with said cleavable signal elements.

### Summary of the invention

The present invention is related to a method for the detection and/or the quantification of a target molecule as described in the claims.

The present invention is also related to a disc having fixed upon its surface a non-cleavable capture molecule as described in the claims, and which can be used in the detection and/or quantification method according to the invention.

Another aspect of the present invention is related to a preparation process of said disc, a diagnostic kit comprising said disc, a diagnostic and reading device comprising said disc or a diagnostic and reading device which allows the reading and the analysis of the data present upon the disc according to the invention.

### Technical characteristics of the "disc"

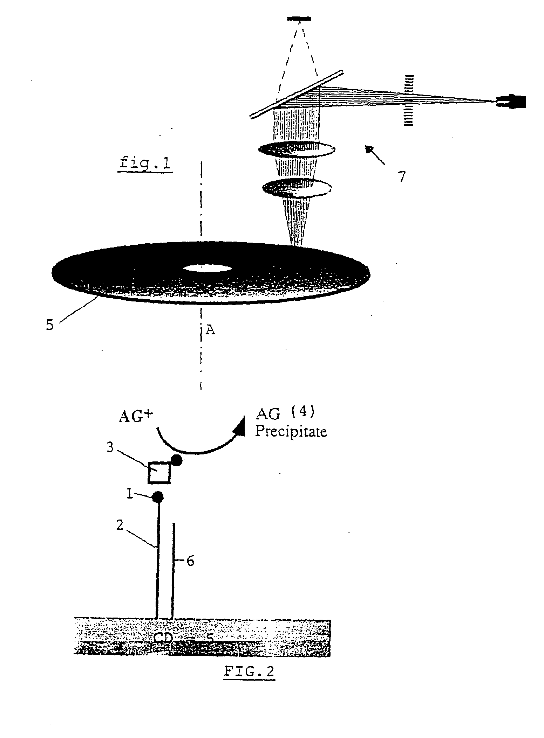

By the term "disc" is meant a flat solid support (usually in the form of a disc) which comprises a hole that allows its rotation according to an axis (A) which is located in the center of said hole), made in a rigid material comprising usually one or more polymer layers and which can be covered by one or more metal layers (like gold or aluminum thin layers) so as to allow penetration and reflection of a light beam, preferably a laser beam, which is used for the detection and the reading of pre-registered data upon said disc (see Fig. 1).

The configuration of said polymeric and metallic layers is prepared in order to allow the penetration and the reflection of the laser beam only upon selected layers. For instance, the disc may comprise a superior layer that allows the penetration of the laser beam, which will be reflected only by a second lower metallic layer.

The definition of a "disc" includes any solid support such as a CD or a "DVD" which comprises data than can be read by a CD-reading device (by penetration and reflection of a laser beam).

It is meant by "data than can be read by a CD-reading device", possibly registered data (i.e. about the characteristics of capture molecules upon specific areas of said solid support) or data used for the treatment of a signal which is the result of a binding between a target and a capture molecules.

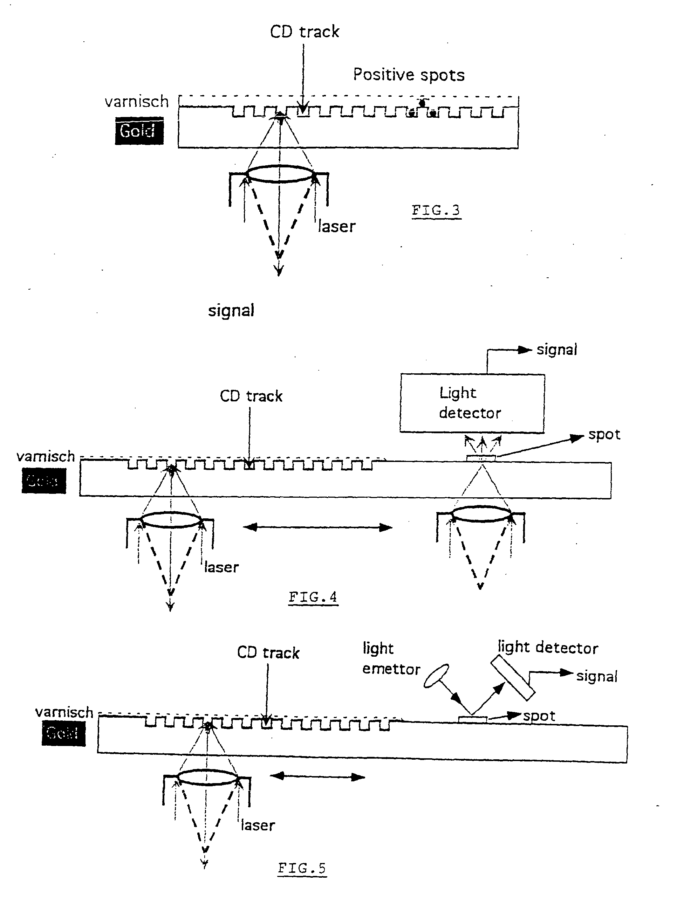

One or more sections of a disc such as a compact-disc are dedicated to data processing by standard read/write digital technology (CD tracks; see Figs. 3 to 5 and 7). Specific data for processing and analysis are recorded on the compact-disc surface using digital recording means. In additional preferred embodiments, read-only memory (ROM) on the disk comprises compact-disc information, instructions, experimental protocols, data analysis and statistical methods that can be accesses by a user operating the disk and recording of the binding location and result between the capture and target molecules.

Additionally, the disc may contain electronic circuitry, including microprocessors for coordination of disc functions, and devices for communication with the disc manipulation and/or reading device or other devices. The disc optimally comprises detectors and sensors, or components of these devices and energy sources for various detection schemes (such as electric power supplies for electrochemical systems, electromagnetic radiation sources for spectroscopic systems), or materials, such as optically-transparent materials, that facilitate operation of and data generation using such detectors and sensors, actuators, communications and data handling devices, mediating communications between the disc and the player/reader device, using electromagnetic (laser, infrared, radiofrequency, microwave), electrical, or other means; circuitry designed for controlling procedures and processes on the disk, including systems diagnostics, assays protocols and analysis of assay data. These are provided in the form of ASICs or ROM which are programmed only at the point-of-manufacture; FGPA's EPROM, flash memory (UV-erasable EPROM), or programmable IC arrays, or similar arrays programmable by the user through the platform manipulation device or other device. Also included in the components of the invention are CPU and microprocessor units and associated RAM operating with an assembler language or high-level language programmable through disk communications, and components for mediating communication with other devices, including facsimile/modem communications with remote display or data analysis systems.

One remarkable aspect of the disc according to the invention is the density of the microscopic array of possibly pre-registered data patterns embedded within the disc materials. It is an optical storage using a laser beam to detect impressions in the surface of the reflective disc. The ability to compress data to such a fine degree and read it back accurately gives the disc according to the invention one of its defining characteristics, the capability of storing huge amounts of data (for a compact-disc of audio data, the amount of storing is around 650 MB of data).

The disc according to the invention could be adapted for the penetration and refection of various laser beams upon various polymeric or metallic layers.

For example, laser devices used for emission of a laser beam and lecture of a reflected laser beam may advantageously comprise a hologram disposed between the disc and a photometre.

The disc is in general of 1.2 mm thick and 4.72 inches in diameter, but smaller supports also exist and could be adapted for specific applications (such as binding between a capture and a target molecules into a Petri dish), and the thickness can be adapted according to the technical requirements of the capture molecule and the detection method of the invention used.

The disc can incorporate grooves to conduct the lecture by a laser beam. In said grooves are incorporated "registered" data that can be thereafter analyzed and advantageously transcripted into digital data. Preferably, said registered data are in the form of binary information. Preferably, these grooves will also comprise fixed non-cleavable capture molecules.

Through its intensive and narrowly focused beam, the laser provides means for precise detection and registration of the passage of thousands of tiny impressions upon the rapidly spinning disc surface. Said detection process generates no friction since the detection is based on the measurement of phase shifts in reflected light. This technique allows the detection of considerable data compaction, since the carefully focused laser beam is able to respond at the speed of the light to extremely small variations in the disc surface.

Light derived from typically natural or artificial sources consists of photons that move in random wave patterns, even when they originate from light beams of the same frequency. Light beams of this sort are considered incoherent, meaning the waves travel in all directions. In comparison, the light associated with lasers is advantageously considered coherent.

A laser beam is created when a source of energy is introduced into what is called an *active medium.* A pair of mirrors positioned on each sides of an active medium are used to channel a portion of the radiation that strikes it. The active medium can consist of a gaseous mix (such as helium and neon) or ions within a crystalline matrix (such as found in the gallium-arsenide lasers typically used in compact-disc (CD) drives and recorders). The materials and the energy source used to stimulate the light determine the strength and intensity of the resulting beam. The lasers used within CD-equipment are usually of extremely low power.

The CD drive laser is directed at the spinning disc and the reflected light passes through a lens and strikes a photodiode (see Figs. 3 to 5). Data on the disc surface is encoded in the form of *pits* (indentations in the disc) and *lands* (the surface of the disc) or disc tracks.

Logic timing circuits coupled to the photodiode can register the difference between the distance the light has traveled (when it strikes the disc surface) and the distance it has traveled (when it strikes an indentation in the disc surface). This difference is detected as a phase shift in the light beam.

As with all digital coded information, the pattern composed of successive pits and lands - relayed as an electronic string of 1's and 0's by the photodiode - can represent much more complex analog equivalents, such as for the present case, the level of the binding between a target and its capture molecule. This information illustrated as pits present on the surface of the disc according to the invention is the result of the binding between the "capture" and "target" molecules.

The disc according to the invention having fixed upon its surface the capture molecule may comprise a protective layer possibly made of organic compounds which allow or improve protection and stabilization of "capture" molecules such as a layer made of proteinic and/or saccharidic compounds like albumin, disaccharides (such as trehalose, etc.).

The composition of such a layer is adapted by the person skilled in the art according to the specific capture molecule used. If necessary, such composition can be adapted in order to allow the laser beam to read through said layer without difficulty and to detect the binding between a "target" molecule and its "capture" molecule or the result of said binding. If necessary, said layer may be omitted before or after the binding between capture and target molecules.

To successfully communicate by means of nothing than a series of pits in a disc requires computer processing and some already available high-technology wizardry. At no point does the laser's read mechanism ever touch the disc surface; all data is preferably conveyed by reflections of the laser. In a normal audio CD, the laser beam takes a certain amount of time to return when it is reflected off the lands, but it takes longer to travel if it is swallowed up and reflected by pits. The depth of the pit is engineered to be 1/4 the wavelength of the laser light. If the reflected beam from the pit cancels out the beam from the land, a signal transition is obtained. Signal transitions (signaled by the beginning or end of a pit) represent binary 1's. If there is no signal transition, this indicates a binary 0.

One particular feature of commercial CD-drives is their property to read such pits and deliver data at unpriseve 900 Kb/sec, making this laser reflector technology particularly suitable for the reading not only of the registered pits but also the result of the binding.

To maintain synchronization while reading the data patterns, the CD drive uses self-clocking mechanism that is commonly found in hard disk drives, which is called *Run Length Limited.* Because data exists within finite divisions on the spiral track, each data division extends approximately 300 nanometers, the CD-microcontroller can produce regular clock signals by synchronizing to the speed of the disc rotation and the occurrences of transitions. Although many forms of data storage use a 8-bit sequence for storing data bytes, the normal CD requires a 14-bit pattern to avoid creating combinations of 1's and 0's that would prevent decoding of the stored data. This modified form of storage is called EFM (Eight-to-Fourteen Modulation). An additional 3 bits called *merging bits* act as separator between the 14-bit part, resulting in a 17-bit pattern to represent a single 8-bit byte of data.

Another significant division of data at the bit level is the *frame,* which consists of 588 bits. The frame encompasses a collection of bits : some of them signify data, others allow the laser to be synchronized with the spinning of the disc and still others contribute to the error-correcting capabilities within the CD equipment. Of this collection of bits only twenty-four 17-bit units (408 bits altogether) can be translated into 8-bits bytes. Many additional bits are needed to convey the information contained in a mere two-dozen data bytes.

The disc according to the invention can be in any "external" shape form. As above-mentioned, the form of said disc is preferably circular or elliptic, but its external shape form may be for instance hexagonal, octagonal, in the form of a square or a triangle which allows the rotation of said disc along a central axis (A).

The disc according to the invention may correspond to the standards of CD-ROM XA, CD-DVD, audio CD, CD-ROM, CD-I, recordable CD and photo or video CD (CD-ROM and CD-I bridge), etc. Said CD standard may differ according to the type of data storage, accuracy and amount of information.

Specific areas of the disc according to the invention can be dedicated to the reading of the reaction that is the result of the binding between the target and the capture molecules. These specific areas are parts of the disc surface according to the invention or an area of the disc on which a second material is fixed and whose surface comprises the capture molecules. These areas can be a cavity in the disc. Said second material is a strip of plastic upon which the binding between the target and the capture molecules has already been performed and which is thereafter fixed upon the disc for its specific reading.

Advantageously, each strip can be loaded with several different capture molecules that will react specifically with the same sample or different samples to be analyzed. Thereafter, the signal can be read individually or simultaneously upon the same disc. A classical disc like a compact-disc could be able to handle 20 or more of such strips.

Preferred embodiments that are most advantageous for manufacturing and operation of the compact-disc of the invention have dimensions within one or more of four pre-existing formats :
- 3-inch compact disk (CD), having a radius of about 3.8 cm and thickness of about 1 mm,
- 5-inch CD, having a radius of about 6 cm and a thickness of 1 mm,
- 8-inch CDV (commercially termed a "Laservision" disk), having a radius of 10 cm and a thickness of 2 mm, and
- 12-inch CDV disk, having a radius of 15 cm and a thickness of 2 mm.

The lifespan of data stored on a magnetic tape when covered by ranges from about 6 to 12 years. Estimates for recordable compact-disc lifespans generally suggest a century of stable data storage.

The lifespan of the specific disc according to the invention is shorter and usually limited by the metal corrosion and the possible denaturation of the capture molecule fixed upon the solid surface. The data stored on CD may exist in the familiar concentric circles (referred to as tracks) of the hard disk drive world or in a continuous spiral like the phonograph records of days past.

One particular property of the compact-disc and its encoded information is the tracking system. Different systems exist in commercially available CD-recorders in order to control the movement of the entire optical pick up in it, radially across the disc, and to search for any one of up to 20000 different radial tracks present on the CD. Said technique can be advantageously adapted for the reading of the signal that is the result of the binding between the target and the capture molecules. The reading of the signal and the reading of the pre-registered information can be done by the same device or by two different reading devices, which can be the same laser beam reading device or two laser beam reading devices.

The correction of the radial tracking (identification of a binding upon a capture molecule by a light beam) is performed by using specific systems, as the one described in the publication The CD-ROM Handbook, 2d Ed. (Chris Sherman Editor, Intertext Publication, McGraw-Hill Inc.). The CD-drives also use special device servomechanisms in order to position the laser's reading head.

Preferably, the disc incorporates microfabricated mechanical and/or optical control components on platforms made from, for example, plastic, silica, quartz, metal or ceramic and/or microchannels as described in the document WO97/21090. For the purposes of this invention, the term "microfabricated" refers to processes that allow production of these structures on the sub-millimeter scale. These processes include but are not restricted to photolithography, etching, stamping and other nano or microtechnological means that are familiar to those skilled in the art.

Additional descriptions of a CD solid support are given in the following publications: The CD-ROM Handbook, 2d Ed. (Chris Sherman Editor, Intertext Publication, McGraw-Hill Inc.), The Complete Recordable CD Guide (Lee Purcell & David Martin, Sybex Editions), Digital Audio and Compact-disc Technology, 2d Ed. (Luc Bart, Luc Theunissen and Guido Vergult, Sony Service Center Europe, Ed. BH Newnes).

### "Target" molecules and "capture" molecules

"Target" and "capture" molecules can be any kind of biological and chemical compounds, which are able to create a binding (or a specific fixation) between each other, said binding or the result of said binding can be detected by a reading device, preferably by using a light beam, preferably a laser beam.

Preferably, said "target" molecule is present in a sample selected from the group consisting of blood, urine, cerebrospinal fluid, plasma, saliva, semen, amniotic fluid, air, water, soil or disrupted biological matter.

Preferably, said "target" and non-cleavable "capture" molecules are synthetic or natural molecules selected from the group consisting of nucleic acids, antibodies, saccharides, lipids, peptides, proteins, lectins, catalysts, receptors, agonists or antagonists of receptors, fluorophores, chromophores, chelates, haptens, ions, molecules having different chiral structures, new synthetic chemical macro-molecules obtained by combinatorial chemistry or other functionalized macrostructures, portions or a combination thereof.

A "non-cleavable capture molecule" means a molecule that does not comprise and need a cleavable spacer as described in the document WO98/01533, to allow or to permit the detection of the binding between a target molecule (or analyte) and a capture molecule. According to the invention, the simple binding between a target and a capture molecules allows thereafter the formation of a signal that was not previously present and that can be detected directly or indirectly by a reading device using a light beam, preferably a laser beam, without requiring any specific cleavage of the capture molecule.

The target or the non-cleavable capture molecules according to the invention are advantageously detected and/or quantified in order to obtain the monitoring, the study and the characteristic behaviors of pathogenic, therapeutical, toxic and/or other improved properties of a target molecule.

The antigens/antibodies binding allows antigens or antibodies detection and are used in diagnostic tests based upon RIA and ELISA detection methods. The ligands/receptors have mainly been developed in pharmacological research for the screening of new molecules (agonists, antagonists or reverse agonists of receptors). Nucleotidic sequences detection has been highly developed through the increased knowledge of the sequence of numerous genes and the development of amplification, hybridization, separation and purification techniques (see, e.g. J. Sambrook, E.F. Fritsch and T. Maniatis, Molecular cloning: laboratory manual, 2nd Ed. Cold Spring, Harbor Laboratory Press, Cold Spring Harbor, New York, 1989).

A first popular detection and amplification method of nucleotide sequences comprises the step of a Polymerase Chain Binding (PCR) (US patents 4,683,195 and 4,683,202) or other amplifications, such as the Ligase Chain Binding (LCR) (Wu and Wallace, 1989, Genomics 4: 560-569), transcription based amplification systems (Kwoh et al. 1989, Proc. Natl. Acad. Sci. USA, 86: 1173-1177) or Cycling Probe Binding (CPR) (Duck et al., 1990, Biotechniques 9: 142-147 and US patent 5,011,769).

Nucleotide sequences detection, quantification and recording by signal of said detection and/or quantification, is obtained after the hybridization of a nucleotide sequence on a capture probe (either by a single or sandwich hybridization) and with the labeling of one of the sequences which give rise to a detection signal, the changes of which can be recorded by the reading device according to the invention.

Many detection methods have been applied to DNA sequences (detected by their own absorbance at 260 nm or by their fluorescence in the presence of ethydium bromide). The use of radioactive labeling like ³²p incorporated into the nucleotidic sequences allows a sensitive detection, but is not recommended for routine assays due to improved safety constraint legislations.

In addition, nucleotide sequences can be labeled by molecules (for example fluoresceine, rhodamine, ruthenium or lanthanide chelate which can be directly detected) or labeled in such a way as to bind enzyme conjugate. A labeling is obtained with the use of biotin or an hapten and an enzyme conjugated to streptavidine or a corresponding antibody. Advantageously, different signals can be obtained according to the product of the binding. For example, peroxidase and alkaline phosphatase give a colored product with the use of TMB (Tetramethylbenzidine) or 5 bromo-4 chloro-3-indolyl-phosphate as substrate. A light emission can be obtained with the use of luminol or AMPPD (3-(2'-spiroadamantane)-4-methoxy-4(3'-phosphoryloxy) 1,2 dioxethane) as substrates.

The DAB (Diaminobenzidine) can be transformed into an insoluble product after oxidation by a peroxidase catalyzed binding. Pyruvate kinase can also be used for the production of ATP which is transformed by luciferase in order to obtain a detected light (bioluminescence detection method).

Advantageously, new technologies like plasmon surface resonance or optical waveguides may be used for the detection of non-labeled target molecules binding and the follow-up of binding kinetic (Gotoh and all. 1995, DNA Res. 2: 285-293, Stimpson et al. 1995, Proc. Natl. Acad. Sci. USA: 92, 6379-6383).

### Fixation of the non-cleavable capture molecule upon the surface of the compact-disc

The non-cleavable capture molecules are preferably fixed at specific intervals on a CD in order to allow specific discrimination between each binding made between a specific non-cleavable capture molecule and its target molecule by a light beam detection device or another device. For specific detection, it is preferred that the capture molecules are located in a specific area of the disc which does not comprise any groove or pre-registered information in order to avoid any false positive which can be the result of a signal upon pre-registered information.

The location of the non-cleavable "capture" molecule on the external surface of the compact-disc can be addressed by conventional physical methods using microlithographic and/or micromachining techniques of incubation which will maintain the non-cleavable capture molecules at certain locations where they will be fixed. An alternative method is obtained by using photoactivable chemical groups which allow the fixation of non-cleavable capture molecules at specific treated locations, such as a portion of said external surface, treated by a light beam (such as a focused laser beam) or treated by selective ion beam or selective plasma treatment (see document WO96/15223).

Advantageously, the external surface of the compact-disc according to the invention also contains data which allows the disc to be read in an laser-based CD reader (information usually stored as a series of pits located in the disc grooves and which are necessary to localize the non-cleavable capture molecule on the surface of the disc). This can be obtained through the presence of appropriated pits or protruding indentations equivalent to the pits in the disc grooves. The addressing (fixation) of the non-cleavable capture molecule on the surface is best obtained using such data and using a laser beam which is part of the overall device and if possible the same laser beam source used for reading the CD information.

The fixation of non-cleavable capture molecules on the disc is obtained using conventional methods based either on covalent or non-covalent bindings. The preferred embodiment is the covalent fixation at one of the molecule extremity, which allows a stable fixation and an homogeneity in the non-cleavable capture molecule presentation at the surface for the binding to the target molecule.

A photoactivable chemical group like azido-nitrophenyl which can be fixed at the extremities of any molecule bearing a free amino group by using for instance heterobifunctional reagent like sulfosuccinimidyl 6-(4'-Azido-2'- Nitrophenylamino hexanoate (Pierce, Rockford, IL, USA). Such a photoactivable group will react only at the place of illumination such as a laser beam (Dontha, N. et al. 1997, Anal. Chem. 69: 2619-2625) and in this way the fixation of a specific probe can be well address on the disc. Other chemical fixations exist like the 5' Phosphate end group fixation of nucleic acid on the amine by carbodiimide or trapping in polypyrrol polymers. Polymeric surfaces can be carboxylated and aminated in order to allow the fixation of most of the biological molecules through bindings well known in organic chemistry (Zammatteo et al. 1996, Anal. Biochem. 236: 85-94).

One particular way to physically address the capture probe is to take advantage of the centripetal force arising from the rotation of the disc. The liquid is projected through inlet pits to enter the disc and then convoyed through microchannels until binding chambers (see International Patent Application WO97/21090).

### Binding between non-cleavable "capture" and "target" molecules

The binding (or fixation) of the target molecule upon its non-cleavable capture molecule is obtained in standard and reproducible conditions which are now well known either for the nucleotide hybridization (hybridization preferably under standard stringent conditions such as the one described by Sambrook et al., §§ 9.31-9.58 in *Molecular Cloning: A Laboratory Manual,* Cold Spring Harbor, Laboratory Press, Cold Spring Harbor, New York (1989)), for the antigen/antibody bindings, for the receptor/ligand binding or other molecules interactions like protein/nucleotides or chemical/chemical molecules recognition.

Preferably, said molecules contain (either by nature or per modification) a functional chemical group (primary amine, sulfhydryl, aldehyde, etc.), a common sequence (nucleic acids), an epitope (antibodies), a hapten or a ligand group, to allow said binding.

The binding between the target molecule and its non-cleavable capture molecule may depend on the specific affinity of the molecules, on the allosteric properties of each molecule, on their ionic environment, on the ionic charge of the molecule and the possible covalent reaction between the target and its non-cleavable capture molecule. Preferably, said conditions are the ones already described in the literature for each type of binding, and can be adapted by the person skilled in the art in order to avoid positive or negative false detections.

Preferably, the optical detection system which can read said binding may comprise a photo-diode which can detect a small light beam and which moves according to a one dimension axe so as to cover the radius of the disc (see Fig. 4). Combined with the rotation of the disc, such focused photo-detection scans the entire surface of the disc and so to assay for the target molecule present at any location on the disc. The preferred detection device is the photo-diode of the commercially available CD readers which are used for music, video or software CD (see Fig. 5).

The photo system can be servocontrolled in order to stay in focus to the detection surface. If a second optical detection system is provided for the detection of the signal, it can also be servocontrolled or linked to the other one for its control, or it may receive from the first one data in order to adjust its focus and its tracks to the disc. The data received from the consecutive reading of the disc surface can also be stored in a computer, reformated if necessary and analyzed for the definition of spots localization.

The photometric signal is obtained once the binding between the target molecule and its non-cleavable capture molecule allows the formation of a photometric signal. Advantageously, the detection and/or the quantification of said binding (binding of the target molecule to its non-cleavable capture molecule) is based upon the principle of CD binary detection system using the variation in the laser beam reflection. A perturbation in the laser reflection is obtained when the laser beam detects in the groove a pit.

According to a first embodiment of the present invention, said pit is advantageously a precipitate which is a result of the binding between the target and the non-cleavable capture molecules.

According to another preferred embodiment, a perturbation in the laser reflection can also be obtained by a corrosive attack upon one or several layers of the compact-disc. For instance, the binding between the target molecule and its non-cleavable capture molecule may provoke a limited modification of the layer which will form an indentation in said layer (see Fig. 3). Such indentations in the layer are usually called "mounds" or "bumps", but are also identified as negative pits (see document Recordable CD Guide, Lee Purcell & David Martin, Sybex Editions). These perturbations will be detected by the laser beam as pits which differ from lands. The binding between the target molecule and its non-cleavable capture molecule can also be detected and quantified directly by a light emission obtained only when said binding takes place.

According to another preferred embodiment of the present invention, the binding of the target molecule upon its non-cleavable capture molecule allows the fixation of one or other molecules which produce a light emission through a chemo, bio, fluoro and/or electroluminescence light system or will create a magnetic and/or electric field which could be detected by specific reading devices 7 (see Fig. 1 and 4).

These systems can be based upon the use of specific enzymes (peroxidase, alkaline phosphatase, pyruvate kinase, etc.) which allow or improve light emission and detection.

One may use a labeled target molecule 6 or a second labeled reactive molecule once the target molecule is captured. This labeled molecule can be the first member of a binding pair such as nucleic acid 2 in a sandwich hybridization assay, using biotin 1 or hapten labeled probes and similarly in an antibody/antigen sandwich binding using similar labeled reactive molecules. After a washing step, the biotin or hapten can react with an enzyme-conjugate streptavidine or antibodies which are then considered as the second member of the binding pair. Enzymes such as peroxidase, alkaline phosphatase, pyruvate kinase or other dehydrogenases can be used.

One selects specific substrate for said enzymes in order to obtain an insoluble product. For example DAB can be oxidized in the presence of peroxidase and form an insoluble product. This product will precipitate upon the non-cleavable capture molecule, and such precipitate will form limps or mounds on the surface of the disc according to the disc face that will be illuminated by the (laser) beam. The reflected (laser) beam intensity will be lowered when illuminating the precipitate and a perturbation in the (laser) reflection can be obtained. Such a perturbation is analyzed by the photosensitive detection device as a pit upon the surface of the disc.

If detected by light transmission through a transparent part of the disc, the presence of the precipitate will show an absorbance that can be measured.

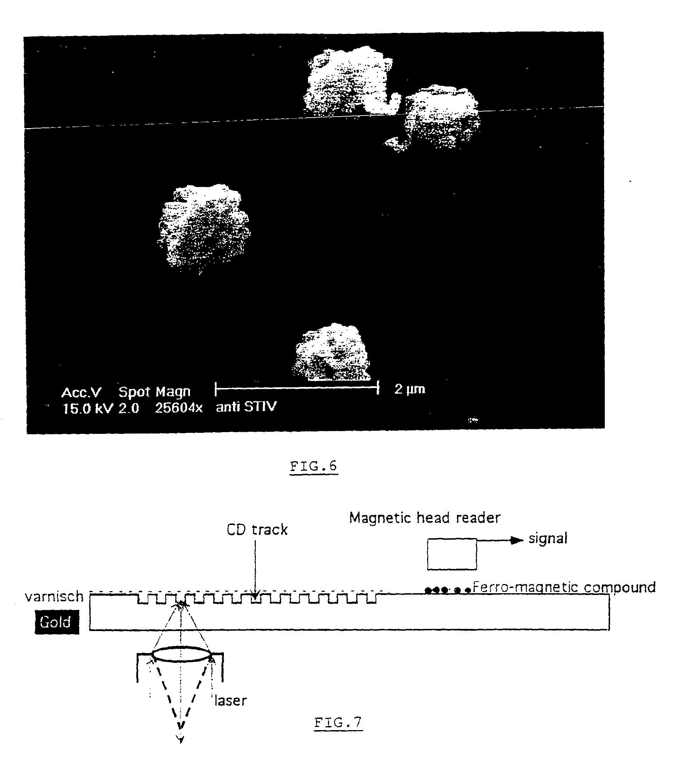

Another insoluble product is obtained when colloidal metal light gold is used, for example bound to streptavidin 3. The colloidal gold catalyzes the reduction of silver (Ag) 4 to form Ag-precipitate when the binding is obtained. Silver deposit can either reduce a (laser) light beam reflection compared to gold or aluminum layers usually present on a disc 5 and can also reflect some of the light if no other metal is present. This precipitate being opaque to the light can also be detected by absorption of the light in a transmission assay through the disc (see Figs. 2 and 6).

It is also possible to use microbeads which bear binding molecules (second binding pair) which allow the recognition of a first binding pair attached to the target molecule. These microbeads will be located at the surface of the disc where the binding of the target molecule and its non-cleavable capture molecule has been obtained. These beads will diffract the (laser) light beam and will create a perturbation in the (laser) reflection. These perturbations in the (laser) reflection will be detected by the photosensitive detection device and analyzed as the pits upon the surface of the disc.

According to another embodiment of the present invention, the detection is obtained by a labeling of the target molecule (with a fluorescent molecule). The (laser) light beam will scan the compact-disc surface and analyze the fluorescence recorded. Many fluorescent molecules associated with the target molecule are available, like fluoresceine, phycoerythrine, rhodamine or lanthanide chelates, which can be easily labeled upon nucleic acids, antibodies or microbeads for a direct or indirect labeling of the target molecule.

The recorded signal can be read either as a binary signal or as an absolute value. The binary signal is advantageously quickly processed as an electronic computerized data and analyzed by appropriate software. This software will convert this information into data which can analyze the detection obtained and quantify the binding between the target molecule and its non-cleavable capture molecule.

Preferably, the disc according to the invention may comprise additional pits, preferably in the groove adjacent to the non-cleavable capture molecule, which give information about the type, the quantity and the specificity of said adjacent non-cleavable capture molecule.

According to a specific embodiment of the present invention, the disc according to the invention bears a fixed oligonucleotide capture probe so as to allow a detection, amplification and possibly quantification of a nucleic acid sequence upon a same solid support (the surface of the disc according to the invention). In an alternative form of execution, the disc comprises fixed PCR primer in order to obtain the production of amplicons and fixation of amplicons upon the external surface of the disc, which allows thereafter their detection (according to the method described by Rasmussen et al. 1991, Anal. Biochem. 198: 138-205).

The disc according to the invention is used in a diagnostic kit, in a diagnostic and reading device which allows automatically the lecture of a sample preparation of a chemical or biological compound, possibly by a previous treatment of said chemical or biological compound (such as genetic amplification of a nucleotide sequence).

Preferably, said device is a system that combines multiple steps or substeps within an integrated system such as an automatic nucleic acid diagnostic system, which allows the steps of purification of the nucleic acid sequences in a sample, their possible amplification (through known genetic amplification methods), their diagnostic and possibly their quantification.

Preferred embodiments of the present invention will be described in the following non-limiting examples.

### Example 1: Detection of DNA on CD

The goal of this experiment was to detect specific DNA by direct hybridization on capture probe bound to CD support. The detection was realized by colorimetric measurement. Capture probe were bound on aminated polycarbonate CD, then hybridization was made with complementary biotinylated DNA and positive hybridization was detected with streptavidin-peroxidase.

### 1. Amination of polycarbonate of CD

CD were first carboxylated by incubation 30 min in NaOH 1N solution at room temperature. After 3 washes with water, carboxylated CD were incubated in a solution of MES 0.1M pH 6 buffer containing water soluble carbodiimide at 1 mg/ml and N-methylpopane 1-3 diamine at 1 mM during 2 hours at room temperature. After 3 washes in MES 0.1M pH 6 buffer and 3 washes with water, the aminated CDs were dried at 37 °C for 30 min.

### 2. Fixation of capture probes on aminated CDs

2 solutions were prepared, one containing CMV capture probe and the other containing HIV capture probe. These solutions were MeIM 0.01 M pH 7.5 buffer containing denatured DNA capture probe (CMV or HIV) at a concentration of 2 µg/ml and carbodiimide at a concentration of 1.6 mg/ml.

3 x 20 µl of these solutions were spotted on two aminated CDs and these CDs were incubated at 50 °C for 5 hours in a wet atmosphere. After three washes of 5 min with NaOH 0.4 N + Tween 0.25% at 50 °C, these CDs were rinsed 3 times with water and dried at 37 °C for 30 min.

### 3. Hybridization of CMV biotinylated DNA on CDs

Both CDs were incubated 5 min in NaOH 0.2 N for denaturating capture probe, then rinsed with 0.1 M maleate buffer pH 7.5 with 0.15 M NaCl. These CDs were then incubated in a hybridization solution containing denatured DNA salmon sperm 100 µg/ml, SSC 4X, Denhardt 5X and denatured CMV biotinylated DNA at a concentration of 70 ng/ml for 2 hours at 65 °C. After hybridization step, the CDs were washed 3 times with 0.01 M maleate buffer containing 15 mM NaCl and Tween 0.3% at room temperature.

The first CD was then incubated with 0.1 M maleate buffer containing 0.15 M NaCl, 0.1% milk powder and streptavidin-peroxidase 1 µg/ml for 45 min at room temperature. After conjugates incubation, both CDs were washed 3 times with 0.01 M maleate buffer containing 15 mM NaCl and Tween 0.3% at room temperature.

### 4. Detection of hybridized DNA

The first CD was then incubated for 10 min in TMB solution (Medgenix). A picture was taken of this CD after 1 min of this incubation to see blue color appearing where positive hybridization occurred (Fig. 4). The result can be obtained by absorption of transmitted light through the CD.

### Example 2: Detection of DNA on CD with maser detection

The DNA capture probe was spotted on the CD surface and the hybridization with the target DNA were identical to the example 1. For the detection of the biotinylated hybridized DNA, the CD was incubated with 0.1 M maleate buffer containing 0.15 M NaCl, 0.1% milk powder and streptavidin-colloidal gold (Sigma, St-Louis, USA) 1 µg/ml for 45 min at room temperature. The CD was further incubated 30 min in a solution made of equal volume of Solution A and B from Silver enhancement kit (Sigma, St-Louis, USA) in order to have silver precipitate where positive hybridization occurred. This CD was recovered with a gold layer to allow a laser CD player to read information written on the CD and to read the interference due to silver precipitate (Fig. 2 and 3).

### Example 3: Detection of protein on CD by light absorption

The CD used were partly inprinted with data on pits and this part was covered with gold. The fixation of the capture molecules was done on the periphery of the CD, directly on the plastic surface.

### 1. Carboxylation of CD

First CDs were incubated 30 min in NaOH 1 N at room temperature then rinsed 3 times with water and dried at 37 °C for 30 min.

### 2. Fixation of antibodies on CDs

Three different types of antibodies were fixed on the carboxylated CD: antibodies against bovine serum albumin, antibodies against fluoresceine (for negative control) and antibodies against streptavidin (for positive control).

20 µl of three different solutions of borate buffer 0.02 M NaCl pH 8.2 containing carbodiimide (Acros) at 1 mg/ml and one type of the three different antibodies at 10 µg/ml were spotted on three different pieces of CD. These spots were incubated overnight at 4 °C, and then rinsed for 10 min with glycine buffer 0.1 M pH 9.2 containing casein at 0.1%, then twice with glycine buffer 0.1 M pH 9.2 containing Tween 20 at 0.1% for 5 min and finally twice with glycine buffer 0.1 M pH 9.2. The CDs were dried at 37 °C during 30 min.

### 3. Detection of bovine serum albumin by ELISA technique on CD

The CDs were incubated at room temperature with the three different antibodies fixed onto the surface with a solution of serum albumin at 10 µg/ml in PBS containing 0.1% of casein. The incubation was for 90 min. The CDs were rinsed 3 times with PBS containing 0.1% of Tween 20, and then incubated with biotinylated antibodies against serum albumin at 20 µg/ml in PBS containing 0.1% of casein for 45 min. They were then rinsed 3 times with PBS containing 0.1% of Tween 20, and then incubated for 45 min the CDs in a solution of PBS containing 0.1% of casein and either Streptavidin-peroxidase at 1 µg/ml. The CDs were rinsed 3 times with PBS containing 0.1% of Tween 20. For detection, the CD where streptavidin-peroxidase was fixed were incubated in a solution of TMB and pictures were taken after 2, 4 and 6 min under camera to see blue color appearing where we had spotted antibodies against BSA and against streptavidin.

### Example 4: Detection of proteins on CD with laser detection

The albumin was spotted on the CD surface and the reaction with the antibodies were identical to the example 3. The conjugate used to react against the biotinylated antibodies was a streptavidin-gold. It. was incubated for 45 min in a PBS solution containing 0.1% casein at a concentration of 1 µg/ml. The streptavidin-gold served as a center for silver reduction. A solution of "silver enhancement" (Sigma) for 15 min at room temperature was used. Silver precipitation was seen at the place where antibodies against BSA and against streptavidin were spotted. A variation in the light absorption was observed, due to the precipitate and the size of the precipitate which are about 1 µm in diameter. The presence of pits was found by reflection of the laser beam (Fig. 5).

### Example 5: Magnetic detection of DNA or protein on CD

Detection of hybridized DNA or protein on CD support can be achieved by magnetic process. Biotin bound to DNA or antibodies can be recognized by streptavidin conjugated to ferro-fluid (Immunicon, Hungtinton Valley, PA, USA). This conjugate is magnetic or paramagnetic according to the size of ferrite nucleus of the ferro-fluid and can then be detected in a magnetic field (Fig. 7).

## Claims

1. Method for the detection and/or the quantification of a target molecule, present in a sample, comprising the steps of :
- allowing a binding between said target molecule and a capture molecule fixed directly upon a side of the surface of a solid support consisting of a compact disk (CD) or DVD comprising registered data that can be read by a CD reading device, said binding resulting in a signal,
- allowing a detection and/or quantification of said signal with the proviso that said signal is not obtained through cleavage of capture molecule, and
- that said signal is read in areas different from areas comprising said registered data and wherein the capture and/or the target molecules are either nucleic acid molecules or proteins, **characterized in that** the capture molecule is located in a specific area of the disk which does not comprise any groove or registered data.

2. Method according to claim 1, **characterized in that** the capture and target molecules are respectively either antigens or antibodies.

3. Method according to claim 1, **characterized in that** the capture and target molecules are respectively either receptors or ligands of said receptors.

4. Method according to claim 1, **characterized in that** the detection and/or the quantification of the signal is obtained by reflection, absorption or diffraction of a light beam, or variation of an electromagnetic field.

5. Method according to claim 4, wherein the light beam is a laser beam.

6. Method according to any one of the preceding claims, **characterized in that** the detection and/or the quantification of the signal is obtained by a fluorescent light emission after excitation of the bound target and capture molecules by a light beam.

7. Method according to any one of the claims 1 to 5, **characterized in that** the detection and/or the quantification of the signal is obtained by a direct emission of a light beam or a change of a magnetic field, which is a result of the binding between the target molecule and its capture molecule.

8. Method according to claim 6 or 7, **characterized in that** the emission of a light beam is generated by a bound molecule which is selected from the group consisting of molecules having chemo, bio, fluoro and/or electroluminescence light.

9. Method according to any one of the preceding claims, **characterized in that** the binding between the target and the capture molecules generates a precipitate upon the surface of the compact disk and/or the corrosion of one or more layer(s) of the surface of the compact disk (CD) or DVD.

10. Method according to the claim 9, wherein said precipitate is an opaque or magnetic precipitate such as a colloidal metal reagent.

11. Method according to the claim 9 or 10, wherein said precipitate is a silver precipitate.

12. Method according to any one of the preceding claims, **characterized in that** the binding between the target and the non-cleavable capture molecules allows the fixation of one or more molecule(s) used in the detection and/or the quantification of a signal which results from said binding.

13. Method according to claim 9 or 12, **characterized in that** the binding between the target and the non-cleavable capture molecule allows the fixation of one or more microbeads or magnetic particles used in the detection and/or the quantification of a signal that results from said binding.

14. Method according to any one of the preceding claims, **characterized in that** the signal is obtained when the disk is rotating upon its axis (A).

15. Method according to any one of the preceding claims, **characterized in that** the registered data are data to be read and recognized by the CD reader device and are data used in the treatment and in the interpretation of the signal resulting from the binding between the capture and the target molecules.

16. A compact disk (CD) or DVD, comprising registered data that can be read by a CD reading device, **characterized in that** it further comprises, non-cleavable capture molecules fixed in dedicated areas which do not comprise registered data, wherein these dedicated areas being different from areas comprising said readable registered data, wherein the non-cleavable capture molecules are fixed directly upon a side of the surface of the capture of said compact disk and wherein non-cleavable capture molecules allow a binding with target molecules to be detected and/or quantified and the target molecules are either nucleic acids or proteins.

17. The compact disk (CD) or DVD according to claim 16, **characterized in that** the non-cleavable capture molecules are selected from the group consisting of nucleotide sequences, antigens, antibodies, receptors, ligands of receptors or a combination thereof.

18. Preparation process of the compact disk (CD) or DVD according to the claims 16 or 17, **characterized in that** the fixation of non-cleavable capture molecules on the surface of the compact disk (CD) or DVD is obtained through a photoactivation.

19. Preparation process of the compact disk (CD) or DVD according to the claims 16 or 17, which comprises the step of a fixation upon a side of the surface of a disk comprising registered data, of non-cleavable capture molecules at specific dedicated areas different from the areas comprising registered data, through a covalent link between an extremity of the capture molecules and the surface layer of the disk.

20. Diagnostic kit comprising the compact disk (CD) or DVD according to the claim 16 or 17 and the reactants allowing the binding between a target molecule and its capture molecule.

21. Detection and/or reading device, which allows the detection and/or the quantification of the signal which results from the binding between a target molecule present in a sample and its capture molecule, and which comprises the compact disk (CD) or DVD according to the claim 16 or 17 or the kit according to claim 20, and means for the detection and/or quantification of said signal.

22. Detection and/or reading device according to claim 21, being a reading compact disk (CD) device.

23. Detection and/or reading device according to claim 21 or 22, **characterized in that** it comprises a first reading head for the reading of registered data upon the compact disk (CD) or DVD and a second reading head for the detection and/or the quantification of the signal which results from the binding between target molecule and its capture molecule.
